# EUROPÄISCHE PATENTSCHRIFT

(11) **EP 2 694 955 B1**
(45) Veröffentlichungstag und Bekanntmachung des Hinweises auf die Patenterteilung: **09.01.2019**
(21) Anmeldenummer: 12711764.6
(22) Anmeldetag: 12.03.2012
(51) Int. Cl.: G01N 25/14, G01N 27/22, G01N 33/28, E21B 47/06

(54) **DRUCKSONDE ZUM NACHWEIS VON CLATHRATEN UND DEREN VERWENDUNG**
PRESSURE PROBE FOR DETECTING CLATHRATES AND THE USE THEREOF
SONDE DE PRESSION DESTINÉE À DÉTECTER LES CLATHRATES, AINSI QUE SON UTILISATION

(30) Priorität: 02.04.2011 DE 102011015942
(43) Veröffentlichungstag der Anmeldung: 12.02.2014
(73) Patentinhaber: Karlsruher Institut für Technologie, 76131 Karlsruhe (DE)
(72) Erfinder: RAPP, Michael, 76676 Graben-Neudorf (DE); VOIGT, Achim, 76344 Eggenstein-Leopoldshafen (DE); CARVALHO, DOS SANTOS, Mauro, 23050-180 Rio de Janeiro - RJ (BR)
(86) Internationale Anmeldenummer: PCT/EP2012/001090
(87) Internationale Veröffentlichungsnummer: WO 2012/136304

(56) Entgegenhaltungen:
- EP-B1- 2 024 077
- DE-A1- 4 116 322
- DE-A1- 19 644 290
- US-A1- 2004 250 606
- US-B1- 6 449 580
- US-B2- 7 541 004
- YAMAMURO O ET AL: "Dielectric study of KOH-doped acetone and trimethylene oxide clathrate hydrates", JOURNAL OF PHYSICS AND CHEMISTRY OF SOLIDS, PERGAMON PRESS, LONDON, GB, Bd. 54, Nr. 2, 1. Februar 1993 (1993-02-01), Seiten 229-235, XP024603147, ISSN: 0022-3697, DOI: 10.1016/0022-3697(93)90314-H [gefunden am 1993-02-01]
- KLINER J R ET AL: "Determination of synthetic hydrate content in sand specimens using dielectrics", CANADIAN GEOTECHNICAL JOURNAL -REVUE CANADIENNE DE GEOTECHNIQUE, NATIONAL RESEARCH COUNCIL OF CANADA, OTTAWA, CA, Bd. 43, Nr. 6, 1. Juni 2006 (2006-06-01), Seiten 551-562, XP009159687, ISSN: 0008-3674, DOI: 10.1139/T06-022

## Beschreibung

Die Erfindung betrifft eine Drucksonde zum Nachweis von Clathraten und deren Verwendung.

Als *Clathrate* (lat. *clatratus* = vergittert) werden Einschlussverbindungen bezeichnet, wobei ein Gastmolekül in einem Gitter, das aus einem Wirtsmolekül gebildet wird, eingeschlossen ist. Natürlich vorkommende Einschlussverbindungen sind die sog. *Gashydrate,* wobei Gasmoleküle wie Methan-Moleküle, aber auch Kohlendioxid- oder Schwefelwasserstoff-Moleküle, als Gast in ein Molekülgitter aus Wassermolekülen eingelagert werden. Einschlussverbindungen aus Methangas in Wasser (Methan/Wasser) werden auch als *Methanhydrate* oder *Methaneis* bezeichnet.

Die Bildung von Einschlussverbindungen erfolgt im Wasser oder im Meerwasser bevorzugt bei hohen Gaskonzentrationen, niedriger Temperatur und hohem Druck, insbesondere in Sedimenten auf dem Meeresboden, wo durch Zerfallsprozesse bio-organischer Stoffe lokal Methan entsteht. Trotz spezifisch geringerer Dichte der Gashydrate (häufig auch in Form eines Sediment-Gashydrat-Gemischs) sorgt Haftung am Untergrund oder spezifisch schwereres gashydrat-freies Sediment dafür, dass das eingelagerte spezifisch leichtere Gashydrat am Meeresgrund verbleibt. Ursprünglich entdeckt wurden Gashydrate jedoch aufgrund ihrer Eigenschaft, Erdgas-Pipelines in kalten Regionen zu verstopfen.

Der Nachweis des Vorhandenseins von Clathraten erfolgt in der Regel über die Erfassung von Druck-Temperatur-Zeit-Diagrammen der betreffenden Einschlussverbindung (z.B. Methan/Wasser) in Autoklaven (Hochdruckmessungen). Druck-Temperatur-Diagramme sind jedoch häufig ungenau und unterliegen den typischen thermodynamischen Verzögerungen. Die messtechnische Erfassung ist auch nur im Labor (Autoklav), nicht jedoch vor Ort möglich. Darüber hinaus existieren in einigen Fällen keine Druck-Temperatur-Diagramme, z. B. bei THF/Wasser-Chlathraten, da beide Bestandteile Flüssigkeiten sind (siehe Y. Park, Y. N. Choi, S.-H. Yeon, und H. Lee, Thermal Expansivity of Tetrahydrofuran Clathrate Hydrate with Diatomic Guest Molecules, J. Phys. Chem. B, Vol. xxx, No. xx, XXXX; P.W. Wilson, D. Lester, A.D.J. Haymet, Heterogeneous nucleation of clathrates from supercooled tetrahydrofuran (THF)/water mixtures, and the effect of an added catalyst, Chem. Eng. Sc. 60, 2937 - 41, 2005)

Clathrate in Form von Flüssigphasen (z.B. THF/Wasser) lassen sich entweder optisch mittels Laser-Infrarot-Absorption oder über Viskositätsmessungen nachweisen. Optische Untersuchungen erfordern jedoch spezielle optische Fenster, die sich auch bei den extremen Druckbedingungen, die bei der Clathratbildung (z. B. 150 MPa für Methanhydrat) auftreten, einsetzen lassen.

Aus dem Projekt *SUGAR* - Submarine Gashydrat-Lagerstätten, Teilprojekt A2.2, *Aktive Elektromagnetik zur Evaluierung und Quantifizierung von Gashydratvorkommen,* BMBF Förderung Nr. 03G0688A, IFM-GEOMAR, Kiel (http://www.ifm-geomar.de/index. php?id=sugar a11000) ist es bekannt, Hochfrequenz-Messungen mittels Schleppantennen auf dem Meeresgrund vorzunehmen.

Aus der JP 6 058 896 A ist eine Vorrichtung zur Bestimmung der Clathrate von Kohlendioxid mit einem Leitfähigkeitssensor und einem Temperatursensor, die mit einer Temperiereinrichtung in thermischem Kontakt stehen, bekannt.

Die DE 10 2006 022 290 A1 offenbart einen Heizer mit einem integriertem Temperatursensor auf einem Träger, wobei auf einem isolierenden Untergrund eine elektrisch leitfähige Dünnschichtstruktur direkt mit einer elektrisch leitfähigen Dünnschichtstruktur beschichtet ist.

In der DE 10 2009 028 634 A1 ist ein Verfahren zur Herstellung einer Schutzschicht für Widerstandssensoren, die eine metallische Widerstandsschicht aufweisen, beschrieben, wobei das Material der Schutzschicht derart auf die metallische Widerstandsschicht aufgebracht wird, dass die Partikel der Schutzschicht so miteinander verbunden werden, dass sich eine poröse Schutzschicht ausbildet.

Die WO 2004/109807 A2 offenbart in Frage kommende Materialien für eine dielektrische Schicht auf einem Halbleitersubstrat, insbesondere Siliziumdioxid, Siliziumoxinitrid, Diamant, Polymere und poröses Aluminiumoxid.

In der EP 2 024 077 B1 wird eine Vorrichtung beschrieben, mit der sich im Labor wichtige Parameter bei der Herstellung von Clathraten, insbesondere Temperatur, Druck, Leitfähigkeit und Flussrate, aufzeichnen lassen.

Yamamuro et al., "Dielectric study of KOH-doped acetone and trimethylene oxide clathrate hydrates", Journal of physics and chemistry of solids, Pergamon Press, London, GB, Bd. 54, Nr. 2, 1. Februar 1993, Seiten 229-235, ISSN: 0022-3697 offenbart die Nutzung eines Permittivitätssensors zur Messung von Clathraten-Eigenschaften. Kliner et al., "Determination of synthetic hydrate content in sand specimens using dielectrics", Canadian Geotechnical Journal, National Research Council of Canada, Ottawa, CA, Bd. 43, Nr. 6, 1. Juni 2006, Seiten 551-562, ISSN: 0008-3674 offenbart die Messung der Permittivität zum Nachweis von Clathratbildung.

Ausgehend hiervon ist es die Aufgabe der vorliegenden Erfindung, eine möglichst einfach aufgebaute Drucksonde zum Nachweis von Clathraten bereitzustellen, die sich auch vor Ort, z.B. in Pipelines oder auf dem Meeresboden, einsetzen lässt. In einem Aspekt der Erfindung soll die Drucksonde eine Überwachung und Vorhersage von bevorstehender, insbesondere ungewollter Clathratbildung vor Ort an kritischen Stellen, z.B. in über- oder unterseeischen Pipelines und Fördereinrichtungen, ermöglichen.

In einem weiteren Aspekt der Erfindung soll die Drucksonde eine Tiefensondierung zur Prospektion von natürlichen Methanhydrat-Vorkommen in unterseeischen Sedimenten erlauben, wobei es gleichzeitig möglich sein soll, das Auftreten von Methanhydraten in dort vorherrschenden typischen Phasen, wie reinem Meerwasser oder einem Sand/Sediment-Meerwasser-Gemisch, gewährleisten zu können.

In einem weiteren Aspekt der Erfindung soll die Drucksonde schließlich Mittel aufweisen, die es ermöglichen, eine bevorstehende, insbesondere ungewollte Clathratbildung zu detektieren, bevor dies in der Umgebung auftritt.

Diese Aufgabe wird im Hinblick auf die Drucksonde durch die Merkmale des Anspruchs 1 und im Hinblick auf die Verwendungen durch die Merkmale der Ansprüche 5 und 6 gelöst. Die Unteransprüche beschreiben jeweils vorteilhafte Ausgestaltungen der Erfindung.

Die erfindungsgemäße Drucksonde weist eine Vorrichtung zum Nachweis von Clathraten auf, die eine Leiterplatte besitzt, auf der zum einen ein Permittivitätssensor und zum anderen ein Temperatursensor angeordnet sind. Als Leiterplatte dient bevorzugt ein typisches Platinenmaterial, insbesondere eine glasfaserverstärkte Epoxy-Leiterplatte, wie sie üblicherweise in der Elektronik eingesetzt wird. In einer alternativen Ausgestaltung eignet sich als Material für die Leiterplatte auch ein anderes Dielektrikum, vorzugsweise ein Polymer oder eine Keramik.

Der Permittivitätssensor und der Temperatursensor sind jeweils an ihrer Oberfläche mit einer Isolationsschicht versehen, die zur Vermeidung der Messung der Ohmschen Einflüsse der Umgebung dient. Die Isolationsschicht dient zum einen als chemisch resistente Trenneinheit zwischen den Sensoren, einschließlich der zugehörigen Elektronik, auf der einen Seite und der Außenwelt auf der anderen Seite, insbesondere zum Schutz vor Umwelteinflüssen durch Meerwasser, Öl in Pipelines usw., und verhindert so Korrosion auf den Metalloberflächen und Anschlüssen. Zum anderen ist die Isolationsschicht durchlässig für elektromagnetische Wechselfelder und Strahlung im Bereich von 10 kHz und 1 GHz. Bevorzugt besitzt die Isolationsschicht eine Dicke von 1-1000 µm und besteht aus Polyurethan-Lack oder aus Parylen, das sich direkt aus der Gasphase abscheiden lässt. Ebenso sind keramische Substanzen, Glasüberzüge oder Diamantschichten möglich, die bei gleicher Schichtdicke mechanisch robuster sind.

Der Permittivitätssensor weist bevorzugt eine isolierte Interdigitalstruktur aus einem leitfähigen Elektrodenmaterial auf. Als Temperatursensor dient vorzugsweise ein Widerstandssensor, insbesondere ein PT1000. Alternativ eignen sich ein NiCrNi-Thermoelement oder ein Halbleiter-Temperatursensor.

Entscheidend ist, dass der Temperatursensor und der Permittivitätssensor in möglichst gutem thermischem Kontakt miteinander und mit einer Temperiereinrichtung stehen, die bevorzugt in Form einer Heiz- und/oder Kühlplatte ausgestaltet ist und Mittel dazu aufweist, die zu untersuchende Umgebung des Temperatursensors thermisch, insbesondere in zyklischer Variation, zu beeinflussen. Als Heizelement wird bevorzugt ein Heizleiter oder ein Peltier-Element eingesetzt, als Kühlelement dienen bevorzugt ein Peltier-Element oder ein von außen gekühlter Kühlfinger, insbesondere ein externer flüssigkeitsbetriebener Thermostat.

Im Innern der Drucksonde ist eine erfindungsgemäße Vorrichtung so eingebracht, dass sie sich möglichst nahe unter einem in die Wand (Mantel) der Drucksonde eingebrachten Fenster befindet, wobei das Fenster durchlässig für elektromagnetische Strahlung im Bereich von 10 kHz und 1 GHz ist und gleichzeitig eine möglichst hohe thermische Leitfähigkeit besitzt. Bevorzugt weist das Fenster eine Dicke von 10-1000 auf µm und besteht aus einer hierfür geeigneten Keramik.

Hierbei ist das Fenster so ausgestaltet, dass es Teil der Isolationsschicht ist, mit der die Oberflächen des Permittivitätssensors und des Temperatursensors versehen sind.

Eine Steuerelektronik für den Permittivitätssensor, den Temperatursensor und die Temperiereinrichtung befindet sich im Inneren der Drucksonde.

In einer besonders bevorzugten Ausgestaltung ist die Steuerelektronik insbesondere für den Permittivitätssensor, den Temperatursensor und die Temperiereinrichtung innerhalb der Wand der Drucksonde angebracht.

Die erfindungsgemäße Drucksonde eignet sich für die Verwendung in einem Verfahren zur Bestimmung der PhasenübergangsTemperatur von Ausgangslösungen zu Clathraten mit dem Verfahrensschritt:
- Messung der dielektrischen Eigenschaften eines Substanzgemischs mittels des Permittivitätssensors bei angelegtem hochfrequentem Signal bei gleichzeitiger Temperaturmessung mit externer Temperatureinstellung.

Während reines Wasser (z. B. bidestilliertes Wasser) eine sehr hohe Permitivität von ca. 80 besitzt, weisen Clathrate und Wassereis eine Permitivität unterhalb von 10 auf. Hierdurch lässt sich reines Wasser auf der einen Seite von Clathraten und Wassereis auf der anderen Seite unterscheiden. Weisen die gebildeten Clathrate verschiedene Kristallisationsphasen mit jeweils unterschiedlichen Dielektrizitätskonstanten auf, lassen sich auch diese einzeln nachweisen.

Die erfindungsgemäße Drucksonde eignet sich somit zur Untersuchung der Bildung von Clathraten aus ihren Ausgangslösungen. Weiterhin lassen sich damit auch modifizierte Bildungsbedingungen untersuchen, indem die Clathratbildung mittels Zusatzstoffen beeinflusst, insbesondere unterdrückt, verhindert oder katalysiert, wird.

Die erfindungsgemäße Drucksonde eignet sich weiterhin für die Verwendung in einem Verfahren für die vorzugsweise stationäre Überwachung und Vorhersage der Clathratbildung mit den Verfahrensschritten:
- Halten der Temperatur der Drucksonde mittels eines in die Drucksonde integrierten Kühlelements unterhalb der Umgebungstemperatur, jedoch oberhalb des Schmelzpunktes der rein wässrigen Phase bzw. des Taupunktes der reinen Feuchte von Erdgasen in einer Pipeline; und
- Auslösen eines Signals, sobald sich lokal an der Drucksonde einzelne Clathrate ausbilden, deren Vorhandensein nachgewiesen wird.

Auf diese Weise lässt bereits dann ein (Alarm-)Signal auslösen, bevor in der allgemeinen Umgebung der Drucksonde eine Phasenumwandlung zu Clathraten auftritt.

Die erfindungsgemäße Drucksonde eignet sich somit für eine (Alarm-)Überwachung und eine Vorhersage von bevorstehender, insbesondere ungewollter Clathratbildung vor Ort an kritischen Stellen, z.B. in über- oder unterseeischen Pipelines und Fördereinrichtungen. Im Alarmfall könnte nach einer Justage von Betriebsparametern die Umgebung der Drucksonde so verändert (beheizt) werden, dass die bevorstehende Clathratbildung unterdrückt oder verhindert wird.

Die erfindungsgemäße Drucksonde eignet sich schließlich für die Verwendung in einem Verfahren für die vorzugsweise mobile Tiefensondierung insbesondere zur dreidimensionalen Prospektion von natürlichen Methangas-Vorkommen mit dem Verfahrensschritt:
- Zyklisches Ändern der Temperatur der Drucksonde über die Umgebungstemperatur mittels eines in die Drucksonde integrierten Heizelements, wodurch vorhandenes Methaneis in der Umgebung bzw. an der Oberfläche der Drucksonde ebenfalls zyklisch aufgeschmolzen wird und durch den hierbei kurzzeitig entstehenden Wasserfilm auf der Oberfläche der Drucksonde ein Sprung der Permittivität auf ca. 80 auftritt, ausgehend von typischen Werten von 6 - 10 für Vereisungen.

Auf diese Weise erfolgt der Nachweis des Vorhandenseins von Clathraten/Wassereis auf der einen Seite und von Wasser auf der anderen Seite, unabhängig vom jeweiligen Gehalt an Sanden oder Sedimenten. Sind in der unmittelbaren Umgebung der Drucksonde entweder Wassereis oder Clathrat vorhanden, so wird durch die Bildung von Wasser im beschriebenen Temperaturzyklus eine ebenfalls zyklische Änderung der Permittivität hervorgerufen, selbst wenn Schlamm und Sediment zugegen sind. Da jedoch in typischen unterseeischen Sedimenten aus grundsätzlichen Überlegungen kein Wassereis vorhanden sein kann (Die Temperatur liegt oberhalb von 4°C und auf dem Meeresboden herrscht dafür eine zu hohe Dichte. Zusätzlich ist noch eine Absicherung durch die Bedingung, dass die Temperatur der Drucksonde oberhalb des Schmelzpunkts der wässrigen Phase liegen muss, gegeben.), wird somit der Nachweis von des Vorhandensein von Clathraten eindeutig.

Die erfindungsgemäße Drucksonde eignet sich somit zur vorzugsweise mobilen Tiefensondierung mittels Drucksonde zur dreidimensionalen Prospektion von natürlichen Methangas-Vorkommen in Sedimenten am Meeresboden oder ausreichend tiefen Gewässern, insbesondere ab einer Tiefe von 400 m. Der Sensor erlaubt dabei eine Unterscheidung der vier typischen Umgebungszustände auf dem oder im Meeresboden:
- Meerwasser,
- Sand/Sediment-Meerwasser-Gemisch,
- Methanhydrat und
- Sand/Sediment-Methanhydrat-Gemisch im Meerwasser.

Die Erfindung wird im Folgenden anhand von Ausführungsbeispielen und den Figuren näher erläutert. Es zeigen:
- **Fig. 1**: Schematische Darstellung der erfindungsgemäßen Vorrichtung in **(a)** Aufsicht bzw. **(b)** Seitenansicht;
- **Fig. 2**: Schematische Darstellung der Drucksonde, in die eine erfindungsgemäße Vorrichtung eingebracht ist, in **(a)** Aufsicht bzw. im **(b)** Querschnitt;
- **Fig. 3**: Untersuchungen mit bidestilliertem Wasser bei zweimaligem Vereisen und jeweils nachfolgendem Auftauen;
- **Fig. 4**: Untersuchungen während des Einfüllens von Sand;
- **Fig. 5**: Untersuchungen bei der Vereisung eines Sand-Wasser-Gemischs;
- **Fig. 6**: Permittivitätshub als jeweilige Folge von verschiedenen angegebenen Vorgängen.

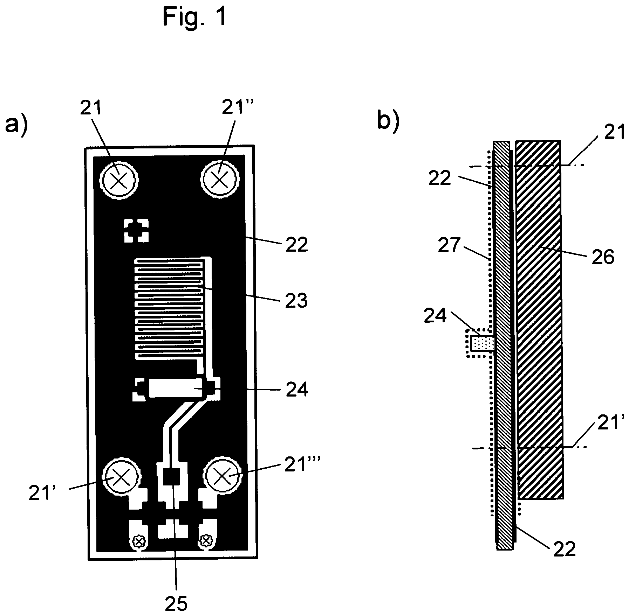

**Fig. 1** zeigt in **(a)** Aufsicht bzw. **(b)** Seitenansicht schematisch eine Vorrichtung **20** zum Nachweis von Clathraten. Ein Permittivitätssensor **23,** der aus einer z.B. mit Parylen isolierten Interdigitalstruktur aus leitfähigem Elektrodenmaterial besteht, wurde auf eine glasfaserverstärkte Epoxy-Leiterplatte (Platine) **22** aufgebracht. Die isoliert angebrachte Interdigitalstruktur diente zur Bestimmung der Permittivitäten (dielektrischen Leitfähigkeit) in der Umgebung des Permittivitätssensors **23** in einem Frequenzbereich von 10 kHz bis 1 GHz. Weiterhin befand sich auf der Leiterplatte **22** ein Temperatursensor **24,** bevorzugt ein PT 1000, der die Temperatur in der zu untersuchenden Umgebung ermittelt.

Der Permittivitätssensor **23** und der Temperatursensor **24** standen in gutem thermischen Kontakt miteinander als auch mit einer vorzugsweise plattenförmigen Temperiereinrichtung **26,** mit deren Hilfe sich die zu messende Umgebung des Permittivitätssensors **23** und des Temperatursensors **24** beeinflussen ließ. Die Temperiereinrichtung **26,** die hier mittels Montagebohrungen **21, 21', 21", 21'''** mit der Leiterplatte **22** verbunden war, bestand aus einer heiz- und kühlbaren Kupferfläche, die sowohl als elektrisches Heizelement als auch als Kühlelement diente und die durch innen liegende Flüssigkeits-Kanäle und einem Haake-Thermostaten thermisch angesteuert werden konnte.

Die Oberfläche der Vorrichtung, einschließlich des Temperatursensors **24** und des Permittivitätssensors **23** mit den Interdigital-Elektroden, war mit einer elektrisch isolierenden und chemisch resistenten Isolationsschicht **27** aus einem Leiterplatten-Schutzlack versehen, die eine Dicke im Bereich von 1-100 µm aufwies.

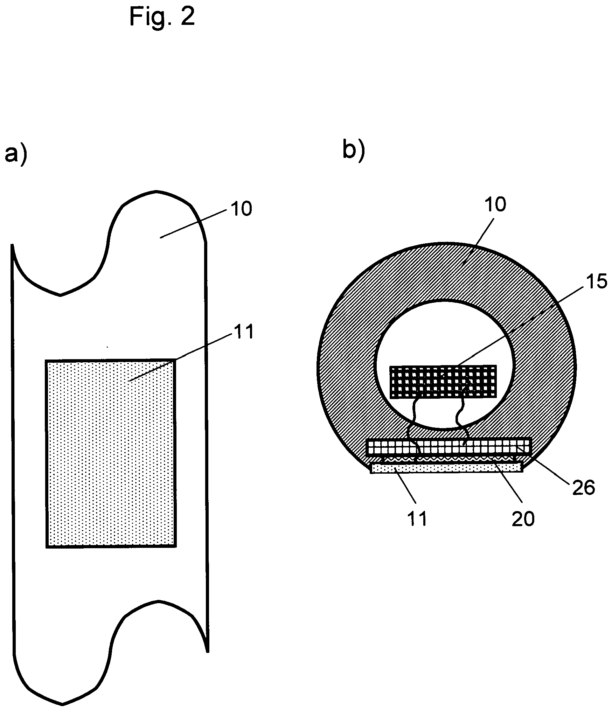

In **Fig. 2** ist schematisch in **(a)** Aufsicht bzw. im **(b)** Querschnitt eine Drucksonde **10** dargestellt, in der die erfindungsgemäße Vorrichtung **20** unmittelbar an der Stelle einer Abflachung hinter einem Fenster **11** integriert ist. Der Permittivitätssensor **23** und der Temperatursensor **24** befinden sich in gutem thermischen Kontakt mit der vorzugsweise plattenförmigen Temperiereinrichtung **26** (Thermostatisierung), die Steuerelektronik **15** ist innerhalb der Drucksonde **10** angebracht. Das Fenster **11** besitzt eine Dicke von 10-100 µm und besteht aus einem elektrisch isolierenden Material, vorzugsweise einer stabilen Keramik, das gleichzeitig als Isolationsschicht **27** dient.

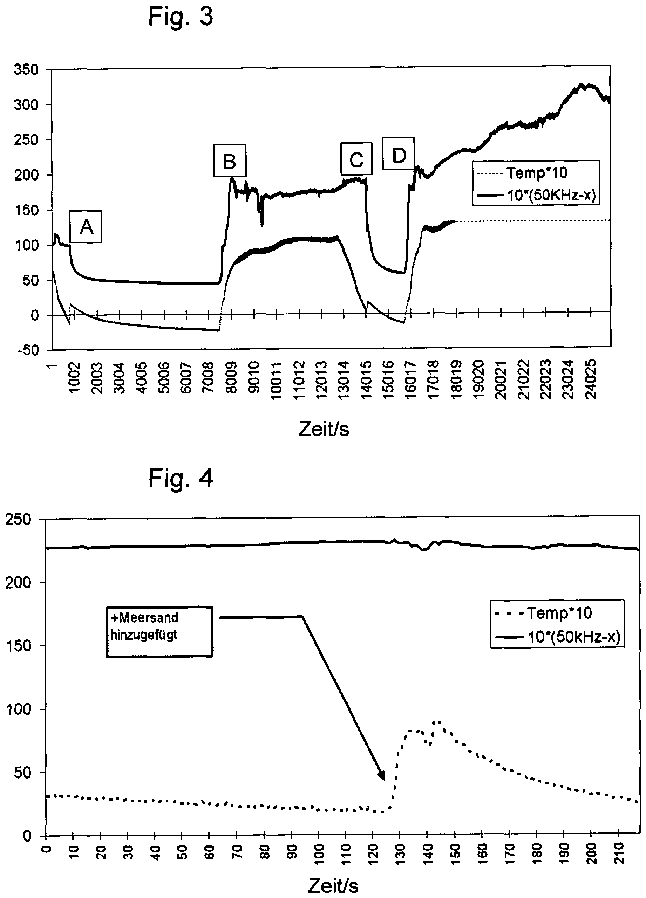

In den **Fig. 3 bis 6** sind Ergebnisse von exemplarisch durchgeführten Versuchen am System Wasser/Wassereis bzw. am System aus Wasser/Wassereis und Sand dargestellt. Hierzu wurde die Vorrichtung in ein Gefäß mit einem Volumen von 100 ml getaucht, das mit bidestilliertem Wasser gefüllt war. Das Gefäß befand sich in einem, dieses aufnehmende, thermisch isolierten Behälter, der mit Leitungswasser und Eiswürfeln gefüllt war, um Messbedingungen um den Gefrierpunkt von Wasser (0°C) zu schaffen.

**Fig. 3** zeigt Untersuchungen mit bidestilliertem Wasser bei zweimaligem Vereisen [A], [C] und jeweils nachfolgendem Auftauen [B], [D]. Start ist kurz vor der ersten Vereisung [A]. Am Temperatursensor **24** ist ein Überschwinger bei 1140s zu erkennen, der eine verzögerte Vereisung bzw. eine latente Wärme anzeigt. Der Permittivitätssensor **23** zeigt ab dem Punkt der tatsächlichen Vereisung, d.h. zeitlich nach dem Peak durch latente Wärme, einen langsamen Abfall zu größeren Oszillationsfrequenzen (durch Differenzbildung in **Fig. 3** als Signalabfall dargestellt), was eine Erniedrigung des Werts der Permittivität bedeutet.

In **Fig. 4** ist der Vorgang des Einfüllens von Sand dargestellt. Die erhöhte Sandtemperatur bewirkt einen Ausschlag des Temperatursensors **24** zu höheren Temperaturen, während der Permittivitätssensor **23** praktisch keinerlei Veränderung zeigt, was zum einen durch seine ausschließliche Sensitivität an der Oberfläche bedingt ist und zum anderen durch die Tatsache, dass Wasser die hohe Permittivität besitzt und nicht ausreichend an der Oberfläche des Sensors verdrängt wird, um eine signifikante Änderung hervorzurufen.

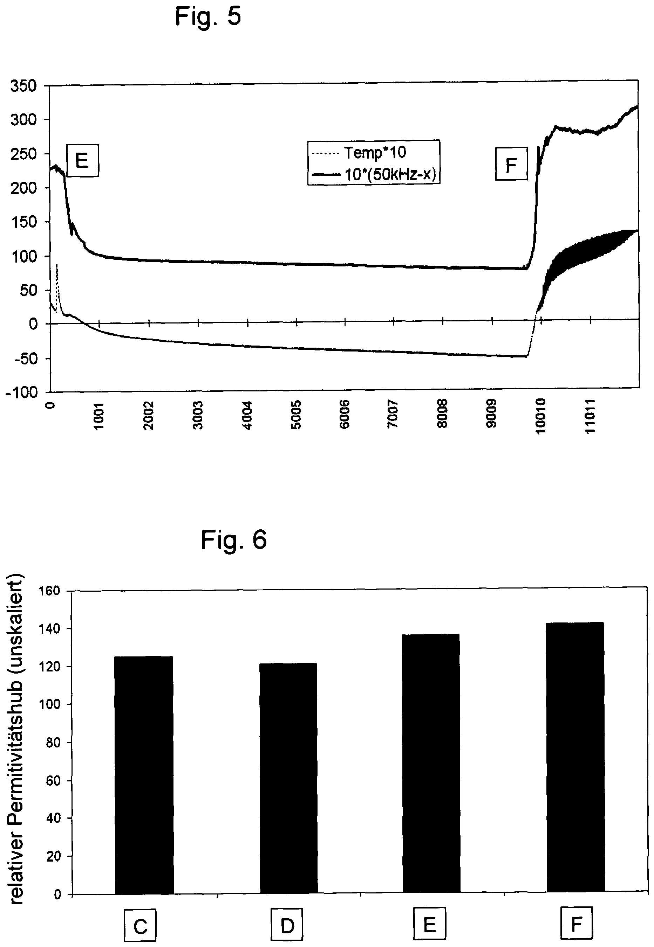

**Fig. 5** zeigt die Vereisung [E] und das anschließende Auftauen [F] eines Sand-Wasser-Gemischs (Schlamm), was sich gut durch den Verlauf des Temperatursensors **24** verfolgen lässt. Eine Ausbildung von latenter Wärme ist hier nicht zu erkennen, vermutlich durch die großen zur Verfügung stehenden Oberflächen an den Sandkörnern, die dem Wasser genügend Keime für eine spontane Vereisung bieten. Der Permittivitätssensor **23** zeigt bei der Vereisung [E] des Gemischs erwartungsgemäß höhere Frequenzen, was durch Differenzbildung in **Fig. 5** als Signalabfall dargestellt ist.

In **Fig. 6** ist zusammenfassend der jeweilige Permittivitätshub als jeweilige Folge der genannten Vorgänge [C] bis [F] dargestellt. Deutlich zu erkennen ist, dass das Signal des Permittivitätssensors **23** im Gegensatz zu einem (nicht dargestellten) Leitfähigkeitssensor sehr sensitiv auf eine Vereisung bzw.

Clathrate reagiert, jedoch unempfindlich gegenüber dem Unterschied von Wasser zu einem Wasser-Sand-Gemisch bleibt. Auf diese Weise wird der Nachweis von Wassereis/Clathraten in der Kombination des Permittivitätssensors **23** mit dem Temperatursensor **24** eindeutig. Der eindeutige Nachweis des Vorhandenseins von Clathraten und nicht von Wassereis erfolgt schließlich durch die oben genannte Überlegung, dass in typischen Sedimenten am Meeresboden oder in tiefen Gewässern kein Wassereis vorhanden sein kann.

## Patentansprüche

1. Drucksonde (10) zum Nachweis von Clathraten, mit einem Mantel und einem in dem Mantel eingebrachten Fenster (11), in deren Innern eine Vorrichtung mit einer Leiterplatte (22), einem Permittivitätssensor (23), einem Temperatursensor (24) und einer Temperiereinrichtung (26) vorhanden ist, wobei
- der Permittivitätssensor (23), der Temperatursensor (24) und die Temperiereinrichtung (26) auf der Leiterplatte (22) aufgebracht und in thermischem Kontakt miteinander sind;
- die Leiterplatte (22) unter dem Fenster (11) angeordnet ist;
- das Fenster durchlässig für elektromagnetische Strahlung im Bereich von 10 kHz und 1 GHz ist, eine möglichst hohe thermische Leitfähigkeit besitzt und Teil einer Isolationsschicht (27) ist, mit der die Oberflächen des Permittivitätssensors (23) und des Temperatursensors (24) versehen sind; und
- eine Steuerelektronik (15) für den Permittivitätssensor (23), den Temperatursensor (24) und die Temperiereinrichtung (26) innerhalb der Drucksonde angebracht ist.

2. Drucksonde (10) nach Anspruch 1, wobei der Permittivitätssensor (23) eine isolierte Interdigitalstruktur aus leitfähigem Elektrodenmaterial aufweist.

3. Drucksonde (10) nach einem der Ansprüche 1 oder 2, wobei die Temperiereinrichtung (26) in Form einer Heiz- und/oder Kühlplatte ausgestaltet ist.

4. Drucksonde (10) nach einem der Ansprüche 1 bis 3, wobei die Isolationsschicht (27) aus einem Polyurethan-Lack, aus Parylen, aus Glas, aus einer Keramik oder aus einer Diamantschicht gebildet ist.

5. Verwendung einer Drucksonde nach einem der Ansprüche 1 bis 4 zur Überwachung, Vorhersage und Unterdrückung von bevorstehender Clathratbildung.

6. Verwendung einer Drucksonde nach einem der Ansprüche 1 bis 4 zur Prospektion von natürlichen Methanhydrat-Vorkommen in Sedimenten am Meeresboden oder in Gewässern.

## Claims

1. Pressure sensor (10) for detecting clathrates, including a casing and a window (11) inserted into said casing, whereby inside said pressure sensor a device with a conduction board (22), a permittivity sensor (23), a temperature sensor (24) and a device for regulating the temperature (26) are present,
**characterized in that**
- the permittivity sensor (23), the temperature sensor (24) and the device for regulating the temperature (26) are placed on the conduction board (22) and are in thermal contact with each other;
- the conduction board (22) is located under the window (11);
- the window is permeable for electromagnetic radiation in the range from 10 kHz to 1 GHz, has the highest possible thermal conductivity and is part of an isolation layer (27), which the surfaces of the permittivity sensors (23) and the temperature sensors (24) are provided with; and
- control electronics (15) for the permittivity sensor (23), the temperature sensor (24) and the device for regulating the temperature (26) are located in the pressure sensor.

2. Pressure sensor (10) according to claim 1, **characterized in that** the permittivity sensor (23) has an isolated interdigital structure comprising a conductive electrode material.

3. Pressure sensor (10) according to one of the claims 1 or 2, **characterized in that** the device for regulating the temperature (26) is in form of a heating and/or cooling plate.

4. Pressure sensor (10) according to one of the claims 1 to 3, **characterized in that** the isolation layer (27) comprises a polyurethane varnish, parylene, glass, a ceramic or a diamond layer.

5. Use of a pressure sensor according to one of the claims 1 to 4 for supervision, prediction and hindrance of the upcoming formation of clathrates.

6. Use of a pressure sensor according to one of the claims 1 to 4 for prospection of natural methanhydrate fields in sediments on the sea floor or in waterbodies.

## Revendications

1. Capteur de pression (10) permettant de détecter des clathrates, muni d'un habillage et d'une fenêtre (11) insérée dans l'habillage,
et à l'intérieur duquel se trouve un dispositif muni d'une plaque de conduction (22), d'un capteur de permittivité (23), d'un capteur de température (24) et d'un dispositif de régulation de température (26),
**caractérisé en ce que**
- le capteur de permittivité (23), le capteur de température (24) et le dispositif de régulation de température (26) sont placés sur la plaque de conduction (22) et sont en contact thermique entre eux;
- la plaque de conduction est placée sous la fenêtre (11);
- la fenêtre est transparente aux rayonnements électromagnétiques dans la gamme de 10 kHz à 1 GHz et possède une conductivité thermique la plus élevée possible et fait partie d'une couche isolante (27), dont les surfaces du capteur de permittivité (23) et du capteur de température (24) sont pourvues et
- une électronique de commande du capteur de permittivité (23), du capteur de température (24) et du dispositif de régulation de température (26) est placée à l'intérieur du capteur de pression.

2. Capteur de pression (10) selon la revendication 1, **caractérisé en ce que** le capteur de permittivité (23) présente une structure interdigitale isolée constituée d'un matériau d'électrode conducteur.

3. Capteur de pression (10) selon l'une des revendications 1 ou 2, **caractérisé en ce que** le dispositif de régulation de température (26) est réalisé sous la forme d'une plaque de chauffage .et/ou de refroidissement.

4. Capteur de pression (10) selon l'une des revendications 1 à 3, **caractérisé en ce que** la couche isolante (27) est formée de laque polyuréthane, de parylène, de verre, d'une céramique ou d'une couche diamant.

5. Utilisation d'un capteur de pression selon l'une des revendications 1 à 4 dans la surveillance, la prévision et le blocage de la formation imminente de clathrates.

6. Utilisation d'un capteur de pression selon l'une des revendications 1 à 5 dans la prospection de gisements naturels d'hydrates de méthane, dans les sédiments de fonds marins ou dans les plans ou cours d'eau.
